# FASCICULE DE BREVET EUROPEEN

(11) **EP 3 294 154 B1**
(45) Date de publication et mention de la délivrance du brevet: **12.12.2018**
(21) Numéro de dépôt: 16729022.0
(22) Date de dépôt: 12.05.2016
(51) Int. Cl.: A61B 17/16

(54) **FRAISE A LAMER**
SCHNEIDGERÄT
CUTTER

(30) Priorité: 12.05.2015 FR 1554239
(43) Date de publication de la demande: 21.03.2018
(73) Titulaire: L.A.R.S. - laboratoire d'application et de Recherche Scientifique, 21560 Arc-sur-Tille (FR)
(72) Inventeur: BRULEZ, Bernard, 52400 Bourbonne Les Bains (FR); BERBEY, Gérard, 21910 Barges (FR); CERULLI, Giuliano, 06123 Perugia (IT)
(74) Mandataire: Oudin, Stéphane
(86) Numéro de dépôt international: PCT/FR2016/051128
(87) Numéro de publication internationale: WO 2016/181083

(56) Documents cités:
- WO-A1-2012/125546
- WO-A2-2008/099187
- DE-A1- 3 840 466
- US-A1- 2011 238 074

## Description

### Domaine technique

La présente invention concerne un instrument chirurgical multi-usage notamment pour la plastie (ou remplacement prothétique) d'un ligament du genou. Le document WO 2008/099187 A2 définit l'état de la technique le plus proche et détermine le préambule de la revendication 1.

### Technique antérieure

Lors d'une rupture ligamentaire, consécutive par exemple à un accident, il est bien connu qu'il peut être nécessaire de procéder au remplacement du ou des ligaments endommagés qui, pour certains d'entre eux, ne peuvent se reconstituer d'eux-mêmes.

La difficulté du remplacement de ce genre de ligament n'est plus liée aux ligaments eux-mêmes puisqu'on connaît maintenant des réalisations tout à fait convenables de ligaments artificiels, mais à des problèmes d'accessibilité limitée du fait de l'anatomie particulièrement encombrée des articulations, ainsi qu'à des problèmes liés aux supports du ligament artificiel, à savoir les éléments anatomiques dans et sur lesquels on souhaite fixer ledit ligament.

En effet, pour fixer et introduire le ligament, il est nécessaire de réaliser des tunnels (perçages) dans certains des éléments anatomiques tels que le fémur et le tibia. Toutefois, compte tenu de la résistance mécanique de ces éléments anatomiques, il est important de réduire au maximum le nombre et les dimensions de ces tunnels, notamment leur diamètre, afin de ne pas fragiliser lesdits éléments anatomiques.

### Exposé de l'invention

La présente invention vise donc à remédier à ces inconvénients en proposant un instrument chirurgical multi-usage, utilisé notamment pour la plastie (ou remplacement prothétique) d'un ligament du genou, de conception compacte, facile à mettre en oeuvre et permettant de n'augmenter que localement le diamètre d'un tunnel réalisé dans un élément anatomique, notamment et principalement au droit de l'extrémité non accessible dudit tunnel débouchant dans une cavité articulaire.

A cet égard, la présente invention a pour objet une fraise à lamer chirurgicale destinée notamment à la plastie (ou remplacement prothétique) d'un ligament du genou comme définie dans la revendication 1.

De manière avantageuse, le tube porte-lame est un tube creux et comporte :
- une lumière le traversant de part en part et apte à recevoir la lame lorsqu'elle est orientée de sorte que son axe longitudinal coïncide avec l'axe longitudinal dudit tube porte-lame, et
- un orifice le traversant de part en part et étant disposé pour que son axe longitudinal soit perpendiculaire au plan longitudinal de symétrie de la lumière et passe par le centre de ladite lumière, ledit orifice étant apte à coopérer avec un rivet pour permettre à la lame de pivoter autour de son axe longitudinal.

La lame est de préférence une plaque de forme globalement rectangulaire dont l'épaisseur est strictement inférieure à l'épaisseur de la lumière du tube porte-lame, la largeur est au plus égale au diamètre D du tube porte-lame et la longueur est strictement supérieure audit diamètre D, et ladite lame comprend en outre un orifice central traversant son épaisseur de part en part et apte à coopérer avec le rivet pour permettre son pivotement autour de l'axe longitudinal dudit orifice.

Selon un mode de réalisation préféré, les moyens de verrouillage comportent une tige dont l'une des extrémités est apte à coopérer avec la lame et un organe de blocage fixé à l'autre extrémité de ladite tige, apte à coopérer avec l'extrémité du tube porte-lame, opposée à celle recevant la lame, pour faire coulisser ladite tige à l'intérieur du tube porte-lame et la bloquer dans une position déterminée.

La lame comporte avantageusement des découpes situées au milieu de ses bords latéraux et longitudinaux.

L'extrémité de la tige est alors apte à coopérer avec l'une des découpes de la lame.

De manière préférée, l'organe de blocage est solidaire de l'extrémité du tube porte-lame par une liaison hélicoïdale et en ce qu'il comporte un capuchon muni d'un taraudage ou filetage apte à coopérer avec un filetage ou taraudage réalisé sur l'extrémité associée du tube porte-lame.

### Description sommaire des figures

D'autres avantages et caractéristiques ressortiront mieux de la description qui va suivre d'une variante d'exécution d'une fraise à lamer chirurgicale en référence aux dessins annexés sur lesquels :
- la figure 1 est une vue d'ensemble d'une fraise à lamer chirurgicale selon l'invention avec sa lame en position "sortie" ;
- la figure 2 est une vue de détail agrandie de la figure 1 ;
- la figure 3 est une vue de détail selon la direction F de la figure 2 ;
- les figures 4 à 6 sont des vues en coupe agrandie et partielles de la fraise à lamer chirurgicale de la figure 1 à différentes étapes de son utilisation.

### Meilleure manière de réaliser l'invention technique

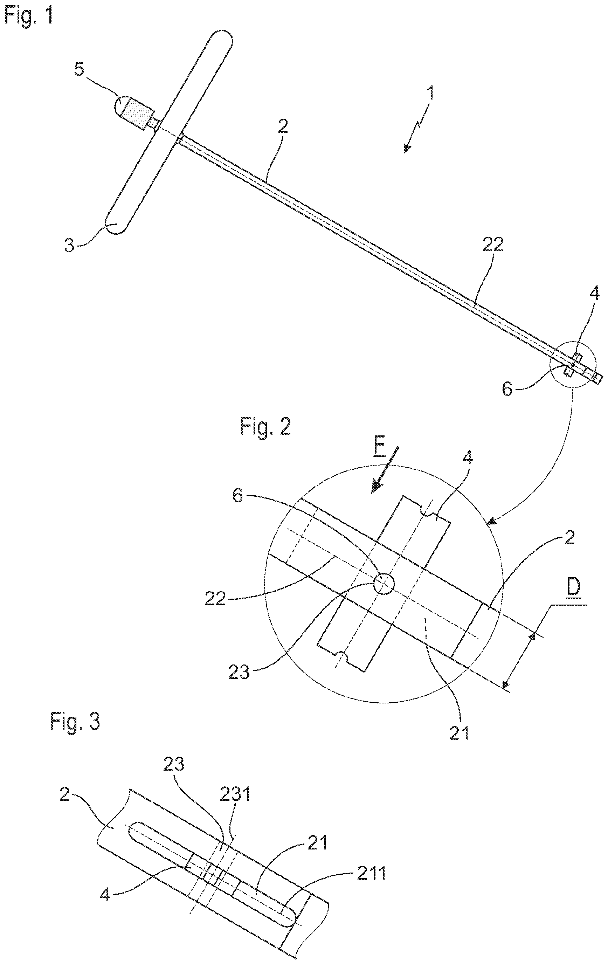

Sur les figures 1 à 6, on a représenté une fraise à lamer 1 chirurgicale, selon l'invention, comportant un tube porte-lame 2 creux muni à l'une de ses extrémités d'une poignée 3, une lame 4 plate de forme globalement rectangulaire montée pivotante à l'autre extrémité dudit tube porte-lame 2 autour d'un axe de rotation perpendiculaire à l'axe longitudinal de ce dernier et des moyens de verrouillage 5 aptes à bloquer en position ladite lame 4.

On désigne ici par "globalement rectangulaire" une forme qui peut être rectangulaire au sens strict avec un angle droit à chaque sommet ou bien une forme rectangulaire avec un angle arrondi à chaque sommet pouvant aller jusqu'à une forme oblongue.

Le tube porte-lame 2, qui s'étend au-delà de la poignée 3, est un tube de section circulaire et comporte :
- une lumière 21 le traversant de part en part et apte à recevoir la lame 4 lorsqu'elle est orientée de sorte que son axe longitudinal coïncide avec l'axe longitudinal 22 dudit tube porte-lame 2, et
- un orifice 23 le traversant de part en part et étant disposé pour que son axe longitudinal 231 soit perpendiculaire au plan 211 longitudinal de symétrie de la lumière 21 et passe par le centre de ladite lumière 21.

Ledit orifice 23 est apte à coopérer avec un rivet 6 pour permettre à la lame 4 de pivoter autour de son axe longitudinal 231.

La lame 4 est une plaque de forme globalement rectangulaire dont l'épaisseur est strictement inférieure à l'épaisseur de la lumière 21 du tube porte-lame 2, la largeur est au plus égale au diamètre D du tube porte-lame 2 et la longueur est strictement supérieure audit diamètre D.

Le tube porte-lame pourra être de section non circulaire sans sortir du cadre de la présente invention, le diamètre D correspondra alors à la dimension dudit tube porte-lame 2 située dans le plan longitudinal de symétrie de ladite lumière.

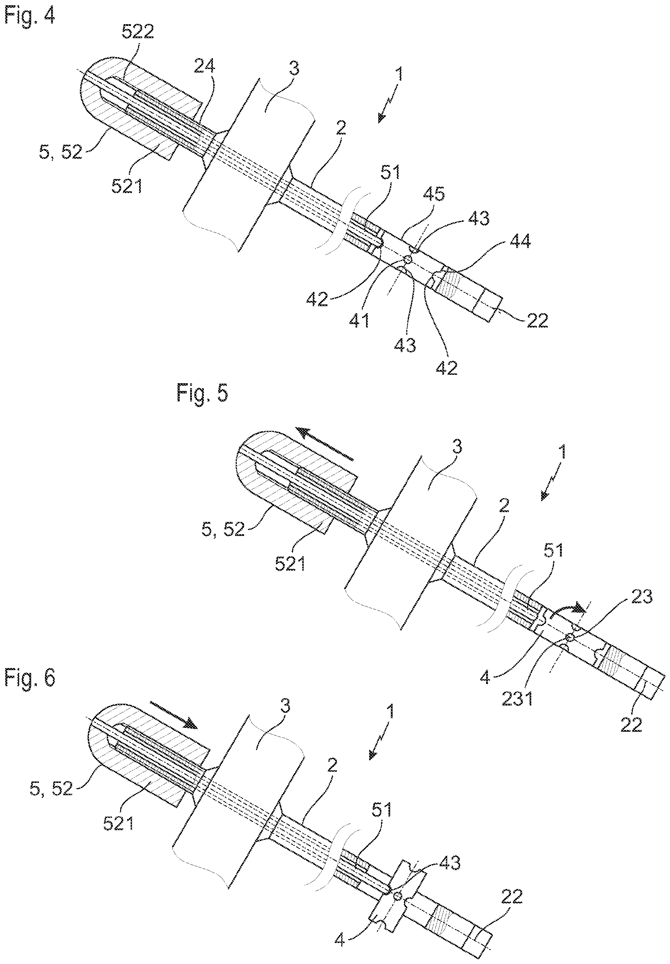

Ladite lame 4 comprend en outre un orifice central 41 traversant son épaisseur de part en part et apte à coopérer avec le rivet 6 pour permettre son pivotement autour de l'axe longitudinal 231 dudit orifice 23 entre une position "rentrée" dans laquelle la lame 4 est orientée de sorte que son axe longitudinal coïncide avec l'axe longitudinal 22 dudit tube porte-lame 2 (Cf. figure 4) et une position "sortie" dans laquelle la lame 4 est orientée de sorte que son axe longitudinal soit perpendiculaire audit axe longitudinal 22 (Cf. figure 6), la lame 4 ne dépassant du tube porte-lame 2 que lorsqu'elle est dans la position "sortie" dans laquelle elle s'étend de part et d'autre dudit tube porte-lame 2.

Par ailleurs, la lame 4 comporte également des découpes 42,43 situées au milieu respectivement de ses bords latéraux 44 et longitudinaux 45, lesdites découpes 42,43 étant de préférence de forme hémicirculaire. Toutefois, lesdites découpes 42,43 pourront être d'une toute autre forme telle que, par exemple, carrée, rectangulaire ou encore triangulaire sans sortir du cadre de la présente invention.

La fraise à lamer 1 selon l'invention comporte également des moyens de verrouillage 5 aptes à bloquer ladite lame 4 dans la position "rentrée" ou la position "sortie". Lesdits moyens de verrouillage 5 comportent une tige 51 dont l'une des extrémités est apte à coopérer avec les découpes 42,43 de la lame 4 et un organe de blocage 52 fixé à l'autre extrémité de ladite tige 51, apte à coopérer avec l'extrémité du tube porte-lame 2, opposée à celle recevant la lame 4, pour faire coulisser ladite tige 51 à l'intérieur du tube porte-lame 2 et la bloquer dans une position déterminée.

Ledit organe de blocage 52 est donc solidaire de l'extrémité du tube porte-lame 2 par une liaison permettant le déplacement relatif de l'organe de blocage 52 par rapport au tube porte-lame 2 au moins selon l'axe longitudinal de ce dernier. Pour des raisons de facilité de nettoyage, ladite liaison est de préférence une liaison hélicoïdale et l'organe de blocage 52 est alors un capuchon 521 muni d'un taraudage 522 apte à coopérer avec un filetage 24 réalisé sur l'extrémité associée du tube porte-lame 2.

Le capuchon 521 de l'organe de blocage 52 pourra être muni d'un filetage apte à coopérer avec un taraudage réalisé sur l'extrémité associée du tube porte-lame 2, sans sortir du cadre de la présente invention.

Ainsi, en référence aux figures 4 à 6 et avec cette configuration de la fraise à lamer 1 chirurgicale, selon l'invention, on procède de la manière décrite ci-après pour réaliser un lamage dans un tunnel (ou pré-tunnel) existant afin d'augmenter localement le diamètre dudit tunnel.

Tout d'abord, après avoir effectué un pré-tunnel de diamètre légèrement supérieur au diamètre du tube porte-lame 2 dans le tibia et le fémur, en respectant un bon alignement sans angulation, on va préparer le tunnel fémoral, puis le tunnel tibial.

Pour cela, on introduit, sous contrôle arthroscopique, le tube porte-lame 2 de la fraise à lamer 1, dont la lame 4 est bloquée en position "rentrée" par les moyens de verrouillage 5.

Ensuite quand l'extrémité de la fraise à lamer 1 est dans la cavité articulaire, on continue à l'introduire d'environ une dizaine de millimètres au-delà de l'extrémité du pré-tunnel débouchant dans ladite cavité.

A ce stade, on libère la lame 4 en dévissant un peu le capuchon 521 de l'organe de blocage 52 de l'extrémité associée du tube porte-lame 2 de manière à ce que l'extrémité de la tige 51 ne soit plus engagée dans la découpe 42 associée, classiquement par une rotation dans le sens contraire des aiguilles d'une montre.

Ensuite, à l'aide d'une broche ou éventuellement d'un instrument arthroscopique, on fait pivoter la lame 4 autour de l'axe longitudinal 231 de l'orifice 23 du tube porte-lame 2 jusqu'à ce qu'elle arrive en position "sortie".

Après, on bloque la lame 4 dans cette position en revissant le capuchon 521 de l'organe de blocage 52 sur l'extrémité associée du tube porte-lame 2 de manière à ce que l'extrémité de la tige 51 soit engagée dans la découpe 43 associée, classiquement par une rotation dans le sens des aiguilles d'une montre.

Enfin, on tire vers l'extérieur la fraise à lamer 1, à l'aide de la poignée 3 en effectuant des mouvements de rotation, creusant ainsi le tunnel d'une longueur et d'un diamètre adaptés à la technique opératoire choisie.

Enfin, on débloque à nouveau la lame 4, on la fait pivoter pour la ramener dans sa position "rentrée", on la bloque à nouveau et on la retire du tunnel fémoral.

Si nécessaire, après avoir sélectionné la fraise à lamer, on procède selon la même technique pour réaliser le tunnel tibial aux dimensions adaptées à la technique opératoire choisie.

### Possibilité d'application industrielle

On comprend bien que la fraise à lamer 1, selon l'invention, est adaptée plus particulièrement à la plastie (ou remplacement prothétique) d'un ligament du genou, mais elle peut également être utilisé pour d'autre type d'intervention chirurgicale.

Enfin, il est clair que la présente invention ne se limite pas à la seule forme d'exécution de cette fraise à lamer 1 ; elle en embrasse, au contraire, toutes les variantes de réalisation et d'application respectant le même principe.

## Revendications

1. Fraise à lamer (1) chirurgicale destinée notamment à la plastie ou au remplacement prothétique d'un ligament du genou et comportant :
- un tube porte-lame (2) muni à l'une de ses extrémités d'une poignée (3),
- une lame (4) de forme globalement rectangulaire montée pivotante à l'autre extrémité dudit tube porte-lame (2) autour d'un axe de rotation perpendiculaire à l'axe longitudinal de ce dernier entre une position "rentrée" dans laquelle elle ne dépasse pas de l'encombrement dudit tube porte-lame (2) et une position "sortie", et
- des moyens de verrouillage (5) aptes à bloquer ladite lame (4) en position "rentrée" ou en position "sortie",
ladite fraise à lamer (1) étant **caractérisée en ce que** la lame (4) est libre en rotation lorsqu'elle n'est pas bloquée par les moyens de verrouillage (5) et **en ce qu'**elle s'étend de part et d'autre dudit tube porte-lame (2) lorsqu'elle est dans sa position "sortie".

2. Fraise à lamer (1) suivant la revendication 1, **caractérisée en ce que** le tube porte-lame (2) est un tube creux et comporte :
- une lumière (21) le traversant de part en part et apte à recevoir la lame (4) lorsqu'elle est orientée de sorte que son axe longitudinal coïncide avec l'axe longitudinal (22) dudit tube porte-lame (2), et
- un orifice (23) le traversant de part en part et étant disposé pour que son axe longitudinal (231) soit perpendiculaire au plan (211) longitudinal de symétrie de la lumière (21) et passe par le centre de ladite lumière (21), ledit orifice (23) étant apte à coopérer avec un rivet (6) pour permettre à la lame (4) de pivoter autour de son axe longitudinal (231).

3. Fraise à lamer (1) suivant la revendication 2, **caractérisée en ce que** la lame (4) est une plaque de forme globalement rectangulaire dont l'épaisseur est strictement inférieure à l'épaisseur de la lumière (21) du tube porte-lame (2), la largeur est au plus égale au diamètre D du tube porte-lame (2) et la longueur est strictement supérieure audit diamètre D, et **en ce que** ladite lame (4) comprend en outre un orifice central (41) traversant son épaisseur de part en part et apte à coopérer avec le rivet (6) pour permettre son pivotement autour de l'axe longitudinal (231) dudit orifice (23).

4. Fraise à lamer (1) suivant l'une quelconque des revendications 1 à 3, **caractérisée en ce que** les moyens de verrouillage (5) comportent une tige (51) dont l'une des extrémités est apte à coopérer avec la lame (4) et un organe de blocage (52) fixé à l'autre extrémité de ladite tige (51), apte à coopérer avec l'extrémité du tube porte-lame (2), opposée à celle recevant la lame (4), pour faire coulisser ladite tige (51) à l'intérieur du tube porte-lame (2) et la bloquer dans une position déterminée.

5. Fraise à lamer (1) suivant l'une quelconque des revendications 1 à 4, **caractérisée en ce que** la lame (4) comporte des découpes (42,43) situées au milieu respectivement de ses bords latéraux (44) et longitudinaux (45).

6. Fraise à lamer (1) suivant la revendication 5, **caractérisée en ce que** lesdites découpes (42,43) sont de forme hémicirculaire.

7. Fraise à lamer (1) suivant la revendication 6, **caractérisée en ce que** l'extrémité de la tige (51) est apte à être engagée dans l'une des découpes (42 ou 43) de la lame (4).

8. Fraise à lamer (1) suivant l'une quelconque des revendications 4 à 7, **caractérisée en ce que** l'organe de blocage (52) est solidaire de l'extrémité du tube porte-lame (2) par une liaison hélicoïdale et **en ce que** l'organe de blocage (52) comporte un capuchon (521) muni d'un taraudage (522) ou filetage apte à coopérer avec un filetage (24) ou taraudage réalisé sur l'extrémité associée du tube porte-lame (2).

## Patentansprüche

1. Chirurgischer Senkfräser (1), der insbesondere zur Plastik oder zum prothetischen Ersatz eines Bandes des Knies vorgesehen ist und umfasst:
- ein Klingenträgerrohr (2), das an einem seiner Enden mit einem Griff (3) ausgestattet ist,
- eine Klinge (4) von allgemein rechteckiger Form, die am anderen Ende des Klingenträgerrohrs (2) um eine zur Längsachse dieses letzteren senkrechte Drehachse zwischen einer "eingefahrenen" Stellung, in der sie nicht über den Raumbedarf des Klingenträgerrohrs (2) hinausgeht, und einer "ausgefahrenen" Stellung schwenkbar montiert ist, und
- Verriegelungsmittel (5), die dazu geeignet sind, die Klinge (4) in "eingefahrener" Stellung oder in "ausgefahrener" Stellung zu arretieren,
wobei der Senkfräser (1) **dadurch gekennzeichnet ist, dass** die Klinge (4) frei drehbeweglich ist, wenn sie nicht durch die Verriegelungsmittel (5) arretiert ist, und dadurch, dass sie sich auf beiden Seiten des Klingenträgerrohrs (2) erstreckt, wenn sie sich in ihrer "ausgefahrenen" Stellung befindet.

2. Senkfräser (1) nach Anspruch 1, **dadurch gekennzeichnet, dass** das Klingenträgerrohr (2) ein Hohlrohr ist und umfasst:
- einen Kanal (21), der von einer Seite zur anderen durch dasselbe hindurchgeht und dazu geeignet ist, die Klinge (4) aufzunehmen, wenn dieselbe so ausgerichtet ist, dass ihre Längsachse mit der Längsachse (22) des Klingenträgerrohrs (2) zusammenfällt, und
- eine Öffnung (23), die von einer Seite zur anderen durch dasselbe hindurchgeht und so angeordnet ist, dass ihre Längsachse (231) zur Längssymmetrieebene (211) des Kanals (21) senkrecht ist und durch den Mittelpunkt des Kanals (21) verläuft, wobei die Öffnung (23) dazu geeignet ist, mit einem Niet (6) zusammenzuwirken, um der Klinge (4) zu ermöglichen, um ihre Längsachse (231) herum zu schwenken.

3. Senkfräser (1) nach Anspruch 2, **dadurch gekennzeichnet, dass** die Klinge (4) eine Platte von allgemein rechteckiger Form ist, deren Dicke strikt kleiner als die Dicke des Kanals (21) des Klingenträgerrohrs (2) ist, deren Breite höchstens gleich dem Durchmesser D des Klingenträgerrohrs (2) ist, und deren Länge strikt größer als der Durchmesser D ist, und dadurch, dass die Klinge (4) weiter eine zentrale Öffnung (41) umfasst, die von einer Seite zur anderen durch ihre Dicke hindurchgeht und dazu geeignet ist, mit dem Niet (6) zusammenzuwirken, um ihr Schwenken um die Längsachse (231) der Öffnung (23) herum zu ermöglichen.

4. Senkfräser (1) nach einem der Ansprüche 1 bis 3, **dadurch gekennzeichnet, dass** die Verriegelungsmittel (5) eine Stange (51) umfassen, von der eines der Enden dazu geeignet ist, mit der Klinge (4) zusammenzuwirken, und ein am anderen Ende der Stange (51) befestigtes Arretierglied (52), das dazu geeignet ist, mit dem Ende des Klingenträgerrohrs (2) zusammenzuwirken, das demjenigen, welches die Klinge (4) aufnimmt, gegenüberliegt, um die Stange (51) im Inneren des Klingenträgerrohrs (2) gleiten zu lassen und dieselbe in einer bestimmten Stellung zu arretieren.

5. Senkfräser (1) nach einem der Ansprüche 1 bis 4, **dadurch gekennzeichnet, dass** die Klinge (4) Ausschnitte (42, 43) umfasst, die jeweils in der Mitte ihrer Seiten-(44) und Längskanten (45) liegen.

6. Senkfräser (1) nach Anspruch 5, **dadurch gekennzeichnet, dass** die Ausschnitte (42, 43) von halbkreisförmiger Form sind.

7. Senkfräser (1) nach Anspruch 6, **dadurch gekennzeichnet, dass** das Ende der Stange (51) dazu geeignet ist, mit einem der Ausschnitte (42 oder 43) der Klinge (4) in Eingriff gebracht zu werden.

8. Senkfräser (1) nach einem der Ansprüche 4 bis 7, **dadurch gekennzeichnet, dass** das Arretierglied (52) über eine Schraubverbindung fest mit dem Ende des Klingenträgerrohrs (2) verbunden ist, und dadurch, dass das Arretierglied (52) eine Kappe (521) umfasst, die mit einem Innengewinde (522) oder Außengewinde ausgestattet ist, welches geeignet ist, mit einem Außengewinde (24) oder Innengewinde zusammenzuwirken, das am zugehörigen Ende des Klingenträgerrohrs (2) ausgeführt ist.

## Claims

1. Surgical cutter (1) intended in particular for the plasty or prosthetic replacement of a knee ligament and comprising:
- a blade-holder tube (2) provided with a handle (3) at one of the ends thereof,
- a blade (4) of generally rectangular shape mounted pivotably, at the other end of said blade-holder tube (2), about a rotation axis perpendicular to the longitudinal axis thereof between a "retracted" position wherein it does not protrude from the body of said blade-holder (2), and a "deployed" position, and
- locking means (5) suitable for locking said blade (4) in the "retracted" position or "deployed" position,
said cutter (1) being **characterised in that** the blade (4) rotates freely when it is not locked by the locking means (5) and **in that** it extends on either side of said blade-holder tube (2) when it is in the "deployed" position.

2. Cutter (1) according to claim 1, **characterised in that** the blade-holder tube (2) is a hollow tube and comprises:
- a slot (21) traversing therethrough and suitable for receiving the blade (4) when it is oriented such that the longitudinal axis thereof is aligned with the longitudinal axis (22) of said blade-holder tube (2), and
- an orifice (23) traversing therethrough and being arranged such that the longitudinal axis (231) thereof is perpendicular to the longitudinal plane (211) of symmetry of the slot (21) and passes through the centre of said slot (21), said orifice (23) being suitable for engaging with a rivet (6) to enable the blade (4) to pivot about the longitudinal axis (231) thereof.

3. Cutter (1) according to claim 2, **characterised in that** the blade (4) is a sheet of generally rectangular shape wherein the thickness is strictly less than the thickness of the slot (21) of the blade-holder tube (2), the width is at most equal to the diameter D of the blade-holder tube (2) and the length is strictly greater than said diameter D, and **in that** said blade (4) further comprises a central orifice (41) traversing through the thickness thereof and suitable for engaging with the rivet (6) to enable the pivoting thereof about the longitudinal axis (231) of said orifice (23).

4. Cutter (1) according to any one of claims 1 to 3, **characterised in that** the locking means (5) comprise a shaft (51) wherein one of the ends is suitable for engaging with the blade (4) and a locking member (52) fastened to the other end of said shaft (51), suitable for engaging with the end of the blade-holder tube (2), opposite that receiving the blade (4), in order to slide said shaft (51) inside the blade-holder tube (2) and lock same in a defined position.

5. Cutter (1) according to any one of claims 1 to 4, **characterised in that** the blade (4) comprises cut-out sections (42, 43) situated at the midpoint respectively of the lateral (44) and longitudinal (45) edges thereof.

6. Cutter (1) according to claim 5, **characterised in that** said cut-out sections (42, 43) are semicircular in shape.

7. Cutter (1) according to claim 6, **characterised in that** the end of the shaft (51) is suitable for being engaged in one of the cut-out sections (42 or 43) of the blade (4).

8. Cutter (1) according to any one of claims 4 to 7, **characterised in that** the locking member (52) is rigidly connected to the end of the blade-holder tube (2) by a helical link and **in that** the locking member (52) comprises a cap (521) provided with an internal thread (522) or external thread suitable for engaging with an external thread (24) or internal thread produced on the associated end of the blade-holder tube (2).
